# EUROPEAN PATENT APPLICATION

(11) **EP 3 834 781 A1**
(43) Date of publication of application: **16.06.2021**
(21) Application number: 19847072.6
(22) Date of filing: 09.08.2019
(51) Int. Cl.: A61F 2/966, A61F 2/954

(54) **CONVEYING DEVICE AND CONVEYING SYSTEM FOR CONTROLLING STEP-BY-STEP RELEASE OF STENT**

(30) Priority: 09.08.2018 CN 201810899863; 09.08.2018 CN 201821276305 U; 09.08.2018 CN 201821276778 U; 09.08.2018 CN 201810899886
(71) Applicant: Hangzhou Endonom Medtech Co., Ltd, Hangzhou, Zhejiang 310051 (CN)
(72) Inventor: WANG, Yongsheng, Hangzhou, Zhejiang 310051 (CN); WU, Shichao, Hangzhou, Zhejiang 310051 (CN); LI, Jianmin, Hangzhou, Zhejiang 310051 (CN)
(74) Representative: KASTEL Patentanwälte PartG mbB
(86) International application number: PCT/CN2019/099897
(87) International publication number: WO 2020/030073

(57) **Abstract**

A delivery system for controlling the release of a stent (800) in a stepwise manner, which includes a stent (800) and a delivery device (1000). The delivery device (1000) includes a core assembly (100); an outer sheath (200) which is hollow and mounted around an outer periphery of the core assembly (100) with a delivering gap (210) defined therebetween, the delivering gap (210) having a distal end for receiving the folded stent (800); and a stent restraining assembly (400) which is configured to restrain a released part of the stent (800) to be partly unfolded when the stent (800) is in a partly released state in such a way to restrain an outer diameter of the released part of the stent (800), and to make the stent (800) be fully unfolded when the stent (800) is in a completely released state. The delivery device (1000) facilitates adjust the position of the stent.

## Description

### CROSS REFERENCE TO RELATED APPLICATIONS

The present disclosure claims priorities to Chinese patent application No. 201810899863.5, entitled "DELIVERY DEVICE AND DELIVERY SYSTEM FOR CONTROLLING RELEASE OF STENT IN A STEPWISE MANNER" filed on August 9, 2018, Chinese patent application No. 201821276305.5, entitled "DELIVERY DEVICE AND DELIVERY SYSTEM FOR CONTROLLING RELEASE OF STENT IN A STEPWISE MANNER" filed on August 9, 2018, Chinese patent application No. 201810899886.6, entitled "A DELIVERY DEVICE" filed on August 9, 2018, and Chinese patent application No. 201821276778.5, entitled "A DELIVERY DEVICE" filed on August 9, 2018, each of which is incorporated by reference herein in its entirety.

### TECHNICAL FIELD

The present invention relates to the technical field of medical devices, and particularly to a delivery device and a delivery system for controlling release of a stent in a stepwise manner.

### BACKGROUND

An aortic aneurysm refers to the abnormal local or diffuse expansion of the aortic wall, which compresses the surrounding organs and causes symptoms. Aortic aneurysms can be classified into true aortic aneurysms, pseudoaneurysms, and dissecting aortic aneurysms according to the structures. An aortic aneurysm causes increased pressure in the inner side of the blood vessel, so it expands progressively. If it has been developed for a long time, the aortic aneurysm eventually ruptures, and the larger the aneurysm, the greater possibility for rupture. According to statistics, without surgical treatment, 90% of patients with thoracic aortic aneurysms die within 5 years, and 75% of patients with abdominal aortic aneurysms die within 5 years.

Thoracic aortic endovascular repair (TEVAR) is currently used to treat aortic ectasia lesions such as aortic dissection, aortic penetrating ulcer, aortic intermural hematoma, thoracic aortic aneurysm, and pseudoaneurysm, etc. Since the first case of abdominal aortic endovascular repair (EVAR) was reported to have been used in the treatment of abdominal aortic aneurysms in the 1990s, it has been rapidly developed in just two decades, because of its advantages such as less trauma, shorter operation and hospitalization time, faster postoperative recovery, lower mortality and complication rate, etc.

Expandable stents are often used as treatment devices for TEVAR and EVAR. In order to maintain well conforming with the blood vessel to be repaired, the diameter of the unfolded stent is generally about 10% greater than the diameter of the blood vessel, and the completely released stent presses against the blood vessel and cannot be re-adjusted if the release position is inaccurate. Thus, the surgeon is required to have rich experience and spend more time and energy to make sure an accurate releasing angle of the stent before the stent is released, which is much time-consuming. Therefore, it is necessary to develop a delivery device and a delivery system for controlling the release of the stent in a stepwise manner, which is convenient to adjust the position of the stent.

### SUMMARY

The technical problem to be solved by the embodiments of the present disclosure is, providing a delivery device and a delivery system for controlling the release of the stent in a stepwise manner, which is convenient to adjust the position of the stent, and facilitates time-saving and energy saving.

In order to solve the aforementioned technical problem, embodiments in a first aspect of the present disclosure provide a delivery device for controlling the release of the stent in a stepwise manner, which is configured to deliver a stent and release the stent in a stepwise manner, the delivery device includes:
a core assembly;
an outer sheath, which is hollow and mounted around an outer periphery of the core assembly, with a delivering gap defined between the outer sheath and the core assembly, wherein the delivering gap has a distal end for receiving a folded stent; and
a stent restraining assembly, configured to enable the stent to be partly unfolded so as to restrain an outer diameter of a released part of the stent when the stent is in a partly released state and to enable the stent to be fully unfolded when the stent is in a completely released state.

In one embodiment of the first aspect of the present disclosure, the delivery device further includes a control handle connected with the outer sheath, wherein the control handle controls the outer sheath to move axially relative to the core assembly so as to enable the folded stent to be in a partly released state or in a completely released state.

In one embodiment of the first aspect of the present disclosure, the stent restraining assembly includes at least one control wire, and the control wire enters the delivering gap through a proximal end of the delivering gap and extending to the distal end of the delivering gap, and wherein the control wire has a distal end configured to circumferentially restrain the released part of the stent.

In one embodiment of the first aspect of the present disclosure, the stent restraining assembly further includes a pull-tab secured to a proximal end of the control wire, the pull-tab is configured to apply a force to a proximal end of the delivery device so as to release the restraining of the control wire to the stent.

In one embodiment of the first aspect of the present disclosure, the delivery device further includes a first locking assembly configured to lock the movement of the stent restraining assembly to avoid releasing the restraining of the control wire to the stent unintentionally.

In one embodiment of the first aspect of the present disclosure, a sheath joint is secured to an outer periphery of the outer sheath, and the control handle includes:
a support body, with the sheath joint provided therein, wherein when being subjected to an axial force, the sheath joint moves axially within the support body to drive the outer sheath to move axially;
a fixed handle, mounted outside a distal end of the support body and secured thereto; and
a sliding handle, mounted outside the support body adjacent to a proximal end of the fixed handle, the sliding handle being rotatable at the outside of the support body so as to drive the sheath joint to move axially.

In one embodiment of the first aspect of the present disclosure, an elongated hole is defined on the support body and extends in an axial direction, a tooth block is mounted outside the support body at a position corresponding to the elongated hole, the sheath joint includes a joint body, and an abutment block and a distal protrusion provided on the joint body, the abutment block and the distal protrusion extend through the elongated hole and abut against a proximal end and a distal end of the tooth block, respectively, in such a way to limit an axial movement of the tooth block relative to the sheath joint, the sliding handle is provided with an internal thread, the tooth block is provided with an external thread engaging with the internal thread, and wherein when the sliding handle rotates, the sliding handle drives the sheath joint to move axially through the tooth block, the abutment block and the distal protrusion, and in turn drives the outer sheath to move axially.

In one embodiment of the first aspect of the present disclosure, the sliding handle is axially slidable on the support body, an unlocking button is embedded in the fixed handle, the unlocking button extends to the side of the sliding handle with a hook, and the hook hooks the sliding handle in such a way that the sliding handle is arranged next to the fixed handle and prevented from axially sliding.

In one embodiment of the first aspect of the present disclosure, the delivery device further includes a push-rod, wherein the push-rod has a distal end disposed in the delivering gap, and the push-rod is configured to abut against the stent to prevent the stent from moving towards the proximal end of the delivering gap when the control handle controls the outer sheath to axially move relative to the core assembly towards the proximal end.

In one embodiment of the first aspect of the present disclosure, the delivery device further includes a support tube, wherein the support tube has a distal end disposed in the delivering gap, and the push-rod is disposed in the support tube.

In one embodiment of the first aspect of the present disclosure, the push-rod defines therein a through passage extending in an axial direction thereof, and the control wire enters the through passage from a proximal end thereof and extends out of the through passage from a distal end thereof.

In one embodiment of the first aspect of the present disclosure, the delivery device further includes a pre-embedded guidewire, wherein the pre-embedded guidewire enters the delivering gap through the proximal end of the delivering gap and extends to the distal end of the delivering gap, the pre-embedded guidewire has a distal end configured to enter an inside of the stent from an outside thereof via a fenestration defined on the stent, and the pre-embedded guidewire is configured to guide a branch guidewire to extend out of the stent via the fenestration of the stent.

In one embodiment of the first aspect of the present disclosure, a second locking assembly is provided at a proximal end of the delivery device and configured to lock the pre-embedded guidewire to be prevented from moving.

Embodiments in a second aspect of the present disclosure provide a delivery system for controlling the release of a stent in a stepwise manner, the delivery system includes a stent and a delivery device, wherein the stent includes a tubular membrane and an annular support frame, and the delivery device includes:
a core assembly;
an outer sheath, which is hollow and mounted around an outer periphery of the core assembly, with a delivering gap defined between the outer sheath and the core assembly, wherein the delivering gap has a distal end receiving the folded stent; and
a stent restraining assembly, configured to restrain a released part of the stent to be partly unfolded so as to restrain an outer diameter of the released part of the stent when the stent is in a partly released state, and to enable the stent to be fully unfolded when the stent is in a completely released state.

In one embodiment of the second aspect of the present disclosure, a plurality of connecting members are provided on the tubular membrane and extend from a proximal end of the tubular membrane towards a distal end of the tubular membrane, and the plurality of connecting members are arranged in at least two columns which are circumferentially arranged spaced apart.

In one embodiment of the second aspect of the present disclosure, the stent restraining assembly is controlled to bound at least two columns of the connecting members together to restrain the stent to be partly unfolded when the stent is in the partly released state, and the stent restraining assembly releases the bound to the connecting members so as to make the stent be fully unfolded when the stent is in the completely released state.

In one embodiment of the second aspect of the present disclosure, a fenestration is provided on the tubular membrane, and the fenestration is located on a released part of the tubular membrane when the stent is in the partly released state.

In one embodiment of the second aspect of the present disclosure, the stent restraining assembly includes at least one control wire which enters the delivering gap through a proximal end of the delivering gap and extending to the distal end of the delivering gap, and the control wire has a distal end restraining the released part of the stent circumferentially.

In one embodiment of the second aspect of the present disclosure, the delivery device further includes a control handle connected with the outer sheath, the control handle controls the outer sheath to move axially relative to the core assembly so as to enable the folded stent to be in a partly released state or in a completely released state.

The embodiments of the present disclosure have advantages as follows.

Since the delivery device for controlling the release of stent in a stepwise manner includes a stent restraining assembly, which is used to restrain a released part of the stent to be partly unfolded when the stent is in a partly released state so as to restrain an outer diameter of the released part of the stent, and to make the stent be fully unfolded when the stent is in a completely released state. The released part of the stent would not tightly conform the blood vessel as the released part of the stent is partly unfolded and the outer diameter thereof is relatively small. In this way, when the stent is released to an inaccurate position, there is no resistance between the fully released part of the stent and the blood vessel, the delivery device can easily drive the stent to move. The delivery device in the embodiments is convenient to adjust the position of the stent, which facilitates time-saving and energy saving of the operator.

### BRIEF DESCRIPTION OF THE DRAWINGS

In order to more clearly illustrate the technical solutions of the embodiments according to the present application or the existing technology, drawings used in the description of the embodiments according to the present application or the existing technology will be briefly introduced below. It should be appreciated that the drawings described below merely illustrate some embodiments of the present application, and other drawings may be obtained by those skilled in the art without departing from the scope of the drawings.
FIG. 1 is a perspective view of a delivery device for controlling the release of a stent in a stepwise manner according to an embodiment of the present disclosure.
FIG. 2 is a cross-sectional view of the delivery device for controlling the release of a stent in a stepwise manner according to the embodiment of the present disclosure, viewed from one direction.
FIG. 3 is a cross-sectional view of the delivery device for controlling the release of a stent in a stepwise manner according to the embodiment of the present disclosure viewed from another direction.
FIG. 4 is an enlarged view of a circled portion A of Figure 2.
FIG. 5 is an enlarged view of a circled portion B of Figure 2.
FIG. 6 is an enlarged view of a circled portion D of Figure 3.
FIG. 7 is an enlarged view of a circled portion C of Figure 2.
FIG. 8 is an enlarged view of a circled portion E of Figure 3.
FIG. 9 is a schematic view of the delivery device for controlling the release of a stent in a stepwise manner according to the embodiment of the present disclosure, viewed from one aspect, in which a stent is in a partly released state.
FIG. 10 is a schematic view of the delivery device for controlling the release of a stent in a stepwise manner according to the embodiment of the present disclosure, viewed from another aspect, in which the stent is in a partly released state.
FIG. 11 is a schematic view showing the stent according to the embodiment of the present disclosure in a completely released state.

Reference numbers shown in the drawings refer to:
1000 - delivery device; 100 - core assembly; 110 - inner core; 120 - outer tube; 130 - leading head; 131 - internal lumen; 140 - stent securing assembly; 142 - positioning sleeve; 143 - securing anchor; 150 - outer tube fastener; 160 - inner core securing steel sleeve; 170 - rear releasing knob; 200 - outer sheath; 210 - delivering gap; 220 - sheath joint; 221 - abutment block; 222 - joint body; 223 - distal protrusion; 300 - control handle; 310 - support body; 311 - elongated hole; 320 - fixed handle; 321 - unlocking button; 322 - hook; 323 - button support; 324 - positioning post; 330 - sliding handle; 332 - rotating knob; 333 - inner flange; 334 - friction-reducing protruding ring; 340 - tooth block; 400 - stent restraining assembly; 410 - control wire; 420 - pull-tab; 500 - first locking assembly; 510 - pull-tab fastener; 520 - proximal releasing knob; 610 - push-rod; 611 - through passage; 615 - rear sliding passage; 620 - support tube; 630 - push-rod fastener; 640 - rear fastener; 650 - rear slideway; 670 - luer joint; 680 - outer cover; 710 - pre-embedded guidewire; 720 - second locking assembly; 721 - wire fastener; 722 - fixed annular protrusion; 723 - blocking member; 800 - stent; 810 - tubular membrane; 811 - fenestration 820 - annular support frame; 830 - connecting member; 840-bare stent; 2000 - delivery system; 3001 - TPU flexible tube; 3002 - three-way valve.

### DESCRIPTION OF THE EMBODIMENTS

The technical solutions in the embodiments of the present disclosure will be clearly and completely described below in conjunction with the accompanying drawings in the embodiments of the present disclosure. Obviously, the described embodiments are only a part of the embodiments of the present disclosure, not all of the embodiments. Based on the embodiments of the present disclosure, all other embodiments obtained by those skilled in the art without inventive work shall fall within the protection scope of the present disclosure.

The terms "include", "comprise", "have" and any variations thereof in the specification, claims and drawings of the present disclosure are intended to cover non-exclusive inclusions. For example, a process, method, system, product or device including a series of steps or elements, is not limited to the steps or elements listed herein, but optionally includes other steps or elements that are not listed herein, or optionally further includes steps or elements inherent to such process, method, product or device. Furthermore, the terms "first," "second," and "third," etc. are used to distinguish between different objects and are not used to describe a particular order.

For clarity of description, one end of the delivery device close to the operator is defined as a proximal end of the delivery device, and the other end relatively far from the operator is defined as a distal end of the delivery device; one end of the stent close to the patient's heart is defined as a proximal end of the stent, and the other end relatively far from the heart is defined as a distal end of the stent. The terms "proximal end" and "distal end" for the delivery device and the stent are defined according to different references.

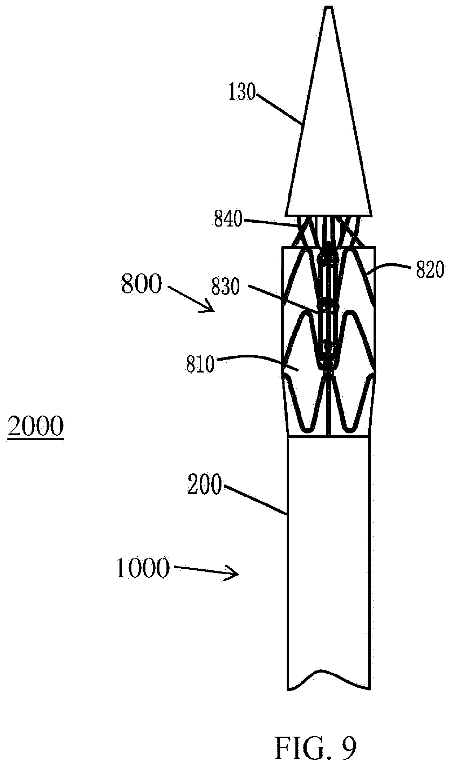

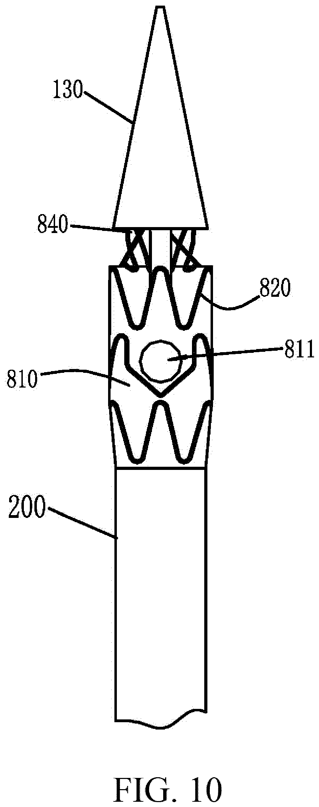

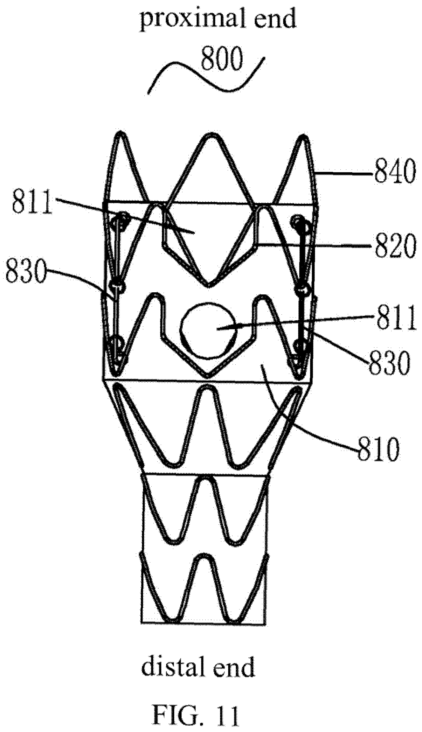

A delivery device for controlling the release of a stent in a stepwise manner is provided according to an embodiment of the present disclosure, which is configured to deliver a stent and control release of the stent in a stepwise manner. Herein, the stent is loaded within the delivery device. The delivery device releases the stent in a step-by-step manner after the stent is delivered to an appropriate location in a blood vessel. Specifically, as shown in FIGs. 9 and 10, a part of the stent 800 is released first from the outer sheath 200. Here, the part of the stent 800 refers to a proximal side of the stent 800, and the remaining part of the stent 800 is remained within the delivery device. Meanwhile, the released proximal side of the stent 800 is still restrained by the delivery device, and the stent 800 is in a partly released state (which will be discussed hereinafter). Since the partly released stent 800 has an outer diameter smaller than the diameter of the blood vessel, it is possible to adjust the position of the stent 800. The remaining part of the stent 800 may be released after the stent 800 has been adjusted in place, at that time, the stent 800 is completely released (as shown in FIG. 11), i.e., in a completely released state. Alternatively, in other embodiment of the present disclosure, the stent 800 may be released in multiple steps, not limited to two steps.

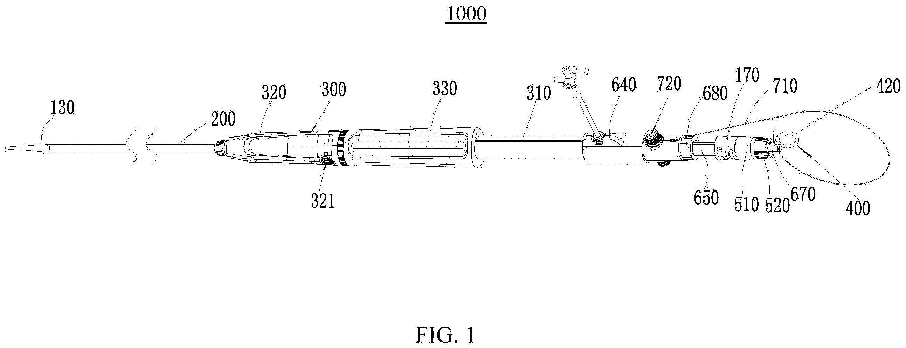

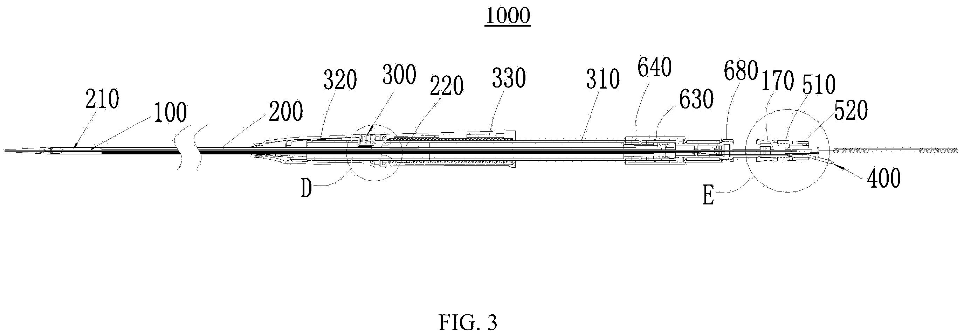

As shown in FIGs. 1 to 3, the delivery device 1000 for controlling the release of a stent in a stepwise manner includes a core assembly 100, an outer sheath 200 and a stent restraining assembly 400.

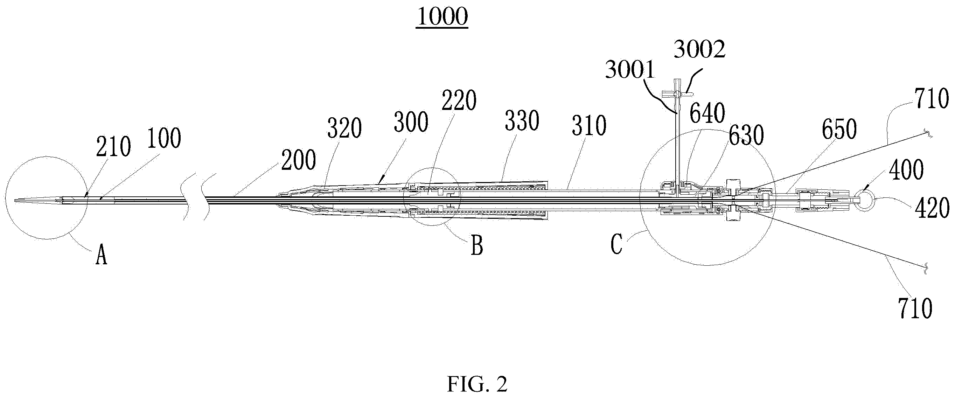

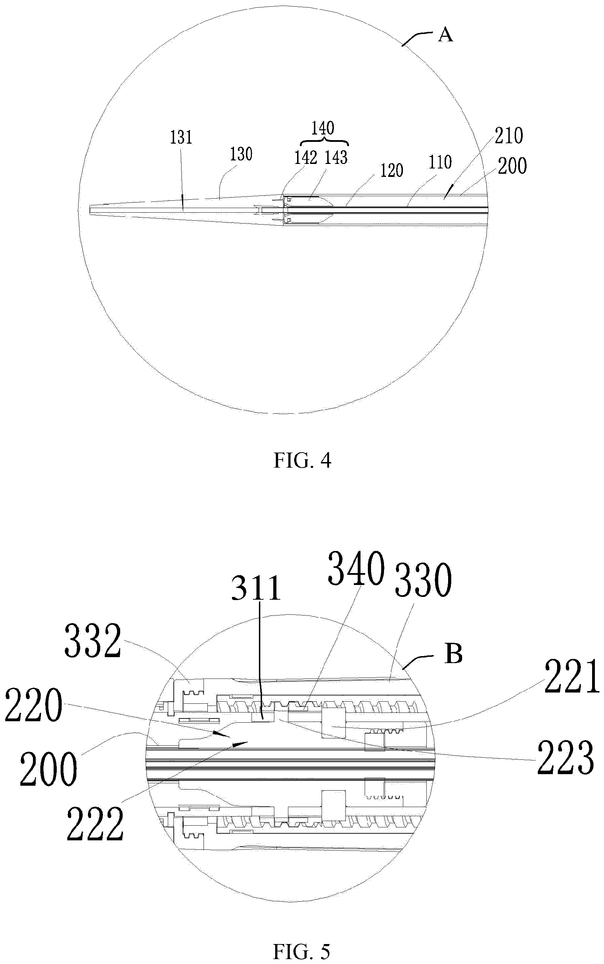

Specifically, in this embodiment, referring to FIGs. 2 and 4, the core assembly 100 includes an inner core 110 and an outer tube 120. The outer tube 120 is hollow and mounted around the inner core 110. The outer tube 120 is slidable relative to the inner core 110 in an axial direction.

In this embodiment, the delivery device 1000 further includes a leading head 130 and a stent securing assembly 140. The leading head 130 is conical and has a distal end being cuspidal. The leading head defines an internal lumen 131 in a center thereof in the axial direction. The inner core 110 has a distal end secured to a proximal end of the leading head 130. The inner core 110 is hollow and communicates with the internal lumen 131 of the leading head 130. The stent securing assembly 140 is provided adjacent to the proximal end of the leading head 130. The stent securing assembly 140 includes a positioning sleeve 142 and a securing anchor 143. The securing anchor 143 is secured to a distal end of the outer tube 120. The securing anchor 143 is columnar and provided with a plurality of protrusions evenly spaced from one another in a circumference thereof for securing a bare stent 840 (as shown in FIG. 11) at a proximal end of the stent 800, so as to position the proximal end of the stent 800. That is, the proximal end of the stent 800 surrounds the securing anchor 143 and is connected thereto. The positioning sleeve 142 has a distal end connected to the leading head 13 by insert molding. The positioning sleeve 142 is hollow and has a proximal end mounted around the securing anchor 143. A limiting gap is defined between the securing anchor 143 and an inner wall of the positioning sleeve 142, and the proximal end of the stent 800 is disposed in the limiting gap.

In this embodiment, the outer sheath 200 is hollow and is mounted around an outer periphery of the core assembly 100. Specifically, the outer sheath 200 is mounted around an outer periphery of the outer tube 120, and is slidable relative to the outer tube 120 in an axial direction. A delivering gap 210 is defined between the outer sheath 200 and the core assembly 100. Specifically, the delivering gap 210 is defined between the outer sheath 200 and the outer tube 120. Herein, the delivering gap 210 refers to a space between an inner wall of the outer sheath 200 and an outer wall of the outer tube 120. The whole securing anchor 143 and the proximal end of the positioning sleeve 142 are disposed in the delivering gap 210. The delivering gap 210 has a distal end for receiving the folded stent 800. Herein, the stent 800 is forced by an external force to have a compressed profile so as to be received in the delivering gap 210. The bare stent 840 at the proximal end of the stent 800 is positioned on the protrusions of the securing anchor 143. The stent 800 is completely received in the delivering gap 210.

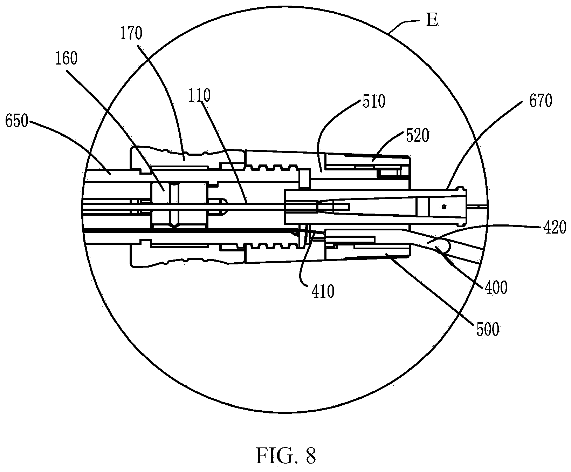

In order to prevent a problem in that a released part of the stent 800 is fully unfolded when the stent 800 is in the partly released state, which results in that the released part of the stent 800 conform the blood vessel tightly (the fully unfolded stent 800 may have a diameter generally about 10% larger than that of the blood vessel) and thus it is impossible to adjust the position of the stent 800 when the stent 800 has been released to an inaccurate position, in this embodiment, as shown in FIGs. 1 to 3 and 8, the stent restraining assembly 400 is provided to restrain the released part of the stent 800 to be partly unfolded when the stent 800 is in the partly released state, so as to restrain the outer diameter of the released part of the stent 800. The released part of the stent 800 refers to a part of the stent 800 not restrained by the outer sheath 200, i.e., the part of the stent 800 which is exposed from the outer sheath 200. Herein, the outer diameter of the released part of the stent 800 when folded is smaller than or equal to 90% of the outer diameter of the released part of the stent 800 when fully unfolded. Since the released part of the stent 800 is partly unfolded and the outer diameter thereof is relatively small, the released part of the stent 800 would not tightly conform the blood vessel. In this way, when the stent 800 is released to an inaccurate position, for example, the stent 800 is inaccurately aligned in a circumferential direction, or a fenestration 811 (as shown in FIG. 10) on the stent 800 is inaccurately positioned, without resistance between the released part of the stent 800 and the blood vessel, the delivery device 1000 can easily drive the stent 800 to move, for example, rotate or move axially, so as to adjust the position of the stent 800 conveniently, for example, to adjust the circumferential alignment of the stent 800. After the stent 800 is adjusted in place, the outer sheath 200 may be operated to move towards the proximal end of the delivery device 100 in the axial direction to completely release the stent 800, and the stent 800 is fully unfolded and tightly conform the blood vessel. The delivery device 1000 in this embodiment can adjust the stent 800 conveniently, which facilitates time-saving and energy saving for the operator. In this embodiment, the stent restraining assembly 400 may make the released part of the stent 800 be fully unfolded as soon as the stent 800 is adjusted in place, or may make the stent 800 be fully unfolded until the stent 800 is in the completely released state. Additionally, in other embodiments of the present disclosure, the delivery device 1000 may be operated to adjust the position of the stent 800 by the operator when the stent 800 is inaccurately positioned in the axial direction.

Still referring to FIGs. 1 to 3, and 8, in this embodiment, the stent restraining assembly 400 includes at least one control wire 410. Herein, one control wire, or two or more control wires 410 may be provided. The control wire 410 may be made of stainless steel. The control wire 410 enters the delivering gap 210 through the proximal end of the delivering gap 210 and extends to the distal end of the delivering gap 210.

As shown in FIG. 11, the stent 800 includes a tubular membrane 810 and an annular support frame 820. A plurality of connecting members 830 are provided on the tubular membrane 810 and extend axially from a proximal end of the tubular membrane 810 towards a distal end of the tubular membrane 810. The connecting members 830 are arranged in at least two columns which are spaced apart in a circumferential direction of the tubular membrane 80. Preferably, the connecting member 830 has a proximal end located at the proximal end of the tubular membrane 810 and a distal end located at the middle of the tubular membrane 810. The tubular membrane 810 is provided with at least one fenestration 811 thereon, which is disposed between the proximal end and the middle of the tubular membrane 810. When the stent 800 is in the partly released state, the tubular membrane 810 is released from its proximal end towards its distal end, but generally not beyond the middle of tubular membrane 810, and at that time the fenestration 811 is located on the released part of the stent 800. The control wire 410 bounds at least two columns of the connecting members 830 together to restrain the stent 800 to be partly unfolded, so as to restrain the outer diameter of the released part of the stent 800. The outer diameter of the released part of the stent 800 is smaller than the diameter of the blood vessel, which facilitates adjust the position of the stent 800, for example, adjust the stent 800 to make the fenestration 811 align with a branch vessel. Preferably, a circumferential length between two columns of the connecting members 410 bounded by the control wire 410 may result in 10% or above reduction of the outer diameter of the released part of the stent 800, that is, the ratio of the outer diameter of the released part of the stent 800 when folded to the outer diameter of the released part of the stent 800 when unfolded is smaller than or equal to 90%.

In this embodiment, as shown in FIGs. 1, 2 and 8, the stent restraining assembly 400 further includes a pull-tab 420 which is secured to a proximal end of the control wire 410, to facilitate handling of the control wire 410 in operation. Specifically, a force applied to the proximal end of the delivery device 1000 by the operator through the pull-tab 420 may release the bound of the control wire 410 to the stent 800, so as to achieve unfolding of the restrained part of the stent 800.

In order to improve security for use of the device, in this embodiment, the delivery device 1000 further includes a first locking assembly 500 configured to lock the movement of the stent restraining assembly 400, so as to avoid releasing the restraining of the control wire 410 to the stent 800 unintentionally. In this embodiment, the first locking assembly 500 is configured to lock the movement of the pull-tab 420, so as to avoid releasing the restraining of the control wire 410 to the stent 800 unintentionally. In addition, in other embodiments of the present disclosure, the first locking assembly 500 may be configured to lock the movement of the control wire 410, so as to avoid releasing the restraining of the control wire 410 to the stent 800 unintentionally. In this embodiment, the first locking assembly 500 includes a pull-tab fastener 510 and a proximal releasing knob 520. The pull-tab fastener 510 is connected to a proximal end of a rear slideway 650 (described hereafter) by a threaded connection. The pull-tab fastener 510 defines a through-hole configured for positioning the pull-tab 420, and a first groove for engaging with the proximal releasing knob 520. The proximal releasing knob 520 is rotatably mounted at a distal end of the pull-tab fastener 510. An L-shaped protrusion is provided inside a distal end of the proximal releasing knob 520, which has a relatively long arm extending in a circumferential direction of the proximal releasing knob 520 and a relatively short arm extending from one end of the relative long arm towards the distal end in an axial direction of the proximal releasing knob 520, so as to define a second groove. A second protrusion is provided at a distal end of the pull-tab 420 and extends outwards. After the pull-tab 420 is inserted in the through-hole and secured therein, the proximal releasing knob 520 is rotated to make the second protrusion enter into the second groove, such that the pull-tab 420 is locked there. In this way, the pull-tab 420 is unable to drive the control wire 410 to move towards the proximal end, so as to avoid releasing the restraining to the stent 800 unintentionally. When the proximal releasing knob 520 is rotated such that the second protrusion disengaged from the second groove, the pull-tab 420 is able to be operated to drive the control wire 410 to move towards the proximal end, so as to release the restraining to the stent 800.

In order to control the release of the stent 800 in a stepwise manner, in this embodiment, as shown in FIGs. 1 and 2, the delivery device 1000 includes a control handle 300 connected with the outer sheath 200. The control handle 300 is configured to control the movement of the outer sheath 200 to move axially relative to the core assembly 100. Specifically, the control handle 300 is configured to control the outer sheath 200 to move axially relative to the outer tube 120. The axial direction refers to a direction extending between the left side and the right side in FIG. 1. With the control handle 300 controlling the outer sheath 200 to move axially, the folded stent 800 in the delivering gap 210 is enabled to be in a partly released state or in a completely released state. Herein, referring to FIGs. 9 and 10, the partly released state refers to a state in which the proximal end of the stent 800 is released and the released part of the stent 800 is no more restrained by the outer sheath 200, in which the position of the stent 800 is adjustable. As shown in FIG. 11, the completely released state refers to a state in which the whole stent 800 is no more restrained by the outer sheath 200, that is an outer periphery of the stent 800 is not surrounded by the outer sheath 200.

In order to adjust an axial position of the outer sheath 200, in this embodiment, referring to FIGs. 1, 2 and 5, the outer sheath 200 is secured to a sheath joint 220. In this embodiment, an outer periphery of a proximal end of the outer sheath 200 is secured to the sheath joint 220. When the sheath joint 220 moves axially, the outer sheath 200 is driven to move axially with the sheath joint 220. The control handle 300 includes a support body 310, a fixed handle 320 and a sliding handle 330. The support body 310 is hollow and the sheath joint 220 is provided therein. When a force is applied to the sheath joint 220 in the axial direction, the sheath joint 220 moves axially within the support body 310 and drives the outer sheath 200 to move axially. The fixed handle 320 is mounted outside a distal end of the support body 310 and secured thereto. The sliding handle 330 is mounted outside the support body 310 and adjacent to a proximal end of the fixed handle 320. The sliding handle 330 is rotatable relative to the support body 310 and drives the sheath joint 220 to move axially. That is, the rotation of the sliding handle 330 is transformed into the axial movement of the sheath joint 220.

Specifically, an elongated hole 311 is defined on the support body 310 and extends in the axial direction thereof. A tooth block 340 is provided outside the support body 310 at a position corresponding to the elongated hole 311. The sheath joint 220 includes a joint body 222, an abutment block 221 and a distal protrusion 223 that are provided on the joint body 222. The abutment block 221 and the distal protrusion 223 extend through the elongated hole 311 and abut against a proximal end and a distal end of the tooth block 340, respectively, so as to limit an axial movement of the tooth block 340 relative to the sheath joint 220. That is, the tooth block 340 is disposed between the abutment block 221 and the distal protrusion 223 in the axial direction. The sliding handle 330 is provided with an internal thread inside, and the tooth block 340 is provided with an external thread outside engaging with the internal thread. When the sliding handle 330 is rotated, the tooth block 340 is driven by the sliding handle 330 to move axially, and thus drives the abutment block 221 and the distal protrusion 223 to move in the axial direction within the elongated hole 311, and in turn the outer sheath 200 is driven to move axially, such that it is possible to enable the stent 800 take the partly released state or take the completely released state.

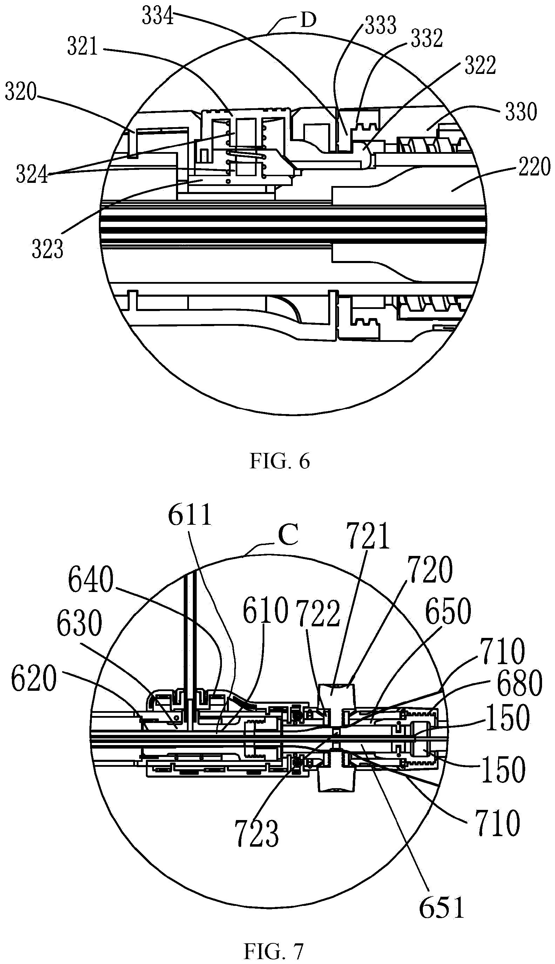

In this embodiment, the sliding handle 330 is further axially slidable on the support body 310, that is, the sliding handle 330 is axially slidable relative to the fixed handle 320. In order to hold the sliding handle 330 axially in position to enhance the security of the operation, in this embodiment, as shown in FIG. 6, an unlocking button 321 is embedded in the fixed handle 320. A hook 322 extends from the unlocking button 321 to the side of the sliding handle 330 . A button support 323 is provided below the unlocking button 321 and secured on the support body 310. Two positioning posts 324 are provided between the unlocking button 321 and the button support 232 and arranged opposite to each other. A spring is mounted around the two positioning posts 324, disposed between the unlocking button 321 and the button support 232, and configured to urge the hook 322 in position. The hook 322 hooks the sliding handle 330, such that the sliding handle 330 is arranged next to the fixed handle 320 and prevented from axially sliding. Specifically, the sliding handle 330 includes a rotating knob 332 disposed at a distal end of the sliding handle 330. The rotating knob 332 has an inner flange 333, and the hook 322 may extend into the rotating knob 332 and engage with the inner flange 333. One side of the inner flange engaging with the hook 322 is defined as an annular positioning groove. That is, the hook 322 is positioned within the positioning groove, such that an axial position of the hook 322 is limited, while allowing the sliding handle 330 to rotate relative to the fixed handle 320. In this embodiment, two friction-reducing protruding rings 334 are coaxially provided on the rotating knob 332. The friction-reducing protruding rings 334 are disposed on a sidewall of a distal end of the rotating knob 322. Due to the friction-reducing protruding ring 332, contact between the fixed handle 320 and the sliding handle 330 is line contact instead of surface contact, which greatly reduces a contact area therebetween and thus reduces friction when they move relative to each other, resulting in a smoother and more accurate release of the stent 800.

In this embodiment, referring to FIGs 1 to 3, and 7, the delivery device 1000 further includes a push-rod 610 and a support tube 620. The support tube 620 is disposed between the outer sheath 200 and the outer tube 120. That is, the support tube 620 is at least partly disposed in the delivering gap 210. The support tube 620 serves to enhance the support. The push-rod 610 is disposed between the support tube 620 and the outer tube 120, with a distal end thereof disposed in the delivering gap 210. When the control handle 300 controls the outer sheath 200 to axially move relative to the core assembly 100 towards the proximal end, the distal end of the push-rod 610 is configured to abut against the stent 800 so as to prevent the stent 800 from moving towards the proximal end of the delivering gap 210. In the meanwhile, the push-rod 610 serves to support the stent 800. In addition, in this embodiment, the push-rod 610 defines a through passage 611 inside in an axial direction. The through passage 611 is configured to allow the control wire 410 and a pre-embedded guidewire 710 to pass therethrough. Specifically, the control wire 410 and the pre-embedded guidewire 710 enter the through passage 611 from a proximal end thereof and extends out of the through passage 611 from a distal end thereof.

In this embodiment, the delivery device 1000 further includes a push-rod fastener 630 and a rear fastener 640. The push-rod fastener 630 is fixedly connected to the proximal ends of the push-rod 610 and the support tube 620. The push-rod fastener 630 has a distal end disposed at an inner side of a proximal end of the support body 310 and secured to the proximal end of the support body 310. The rear fastener 640 is mounted around an outer periphery of the proximal end of the support body 310, and has a distal end secured to the proximal end of the support body 310. In addition, a connecting port extends out from the push-rod fastener 630, which is configured to be connected to an end of a TPU flexible tube 3001. Another end of the TPU flexible tube 3001 is connected with a three-way valve 3002.

In this embodiment, the rear fastener 640 has a proximal end secured to a rear slideway 650 which has a distal end disposed at an inner side of the rear fastener 640. The rear slideway 640 is provided therein with an outer tube fastener 150, which is disposed at a position in proximity to the middle of the rear slideway 650 and secured to an outer periphery of the proximal end of the outer tube 120. An outer cover 680 is provided adjacent to the proximal end of the rear slideway 650 and mounted around the rear slideway 650.

In this embodiment, as shown in FIG. 1 to 3, 7 and 8, an inner core securing steel sleeve 160 and a rear releasing knob 170 are provided at the proximal end of the rear slideway 650. The inner core securing steel sleeve 160 is disposed inside the rear slideway 650. The inner core securing steel sleeve 160 is secured to the inner core 110 and disposed outside the inner core 110. The rear releasing knob 170 is disposed outside the proximal end of the rear slideway 650. The rear releasing knob 170 is secured to the inner core securing steel sleeve 160. An elongated hole is defined at the proximal end of the rear slideway 650 which extends in an axial direction. The rear releasing knob 170 can drive the inner core securing steel sleeve 160 to move in the axial direction within the elongated hole, so as to control a later release of the stent 800. In this embodiment, the proximal end of the rear slideway 650 is secured to the distal end of the pull-tab fastener 510. In this embodiment, the inner core 110 has a proximal end secured to a luer joint 670.

In general, an arterial blood vessel of a human body is connected to a plurality of branch vessels, and when the arterial blood vessel is repaired by the stent 800, the branch vessels sometimes are required to repair simultaneously. In that case, a branch stent is required to enter the branch vessel via the stent 800 to repair the branch vessel. In order to deliver the branch stent to the branch vessel conveniently, in this embodiment, as shown in FIG. 1, 2 and 4, the delivery device 1000 also includes a pre-embedded guidewire 710 that enters the delivering gap 210 through the proximal end and extends to the distal end thereof. The pre-embedded guidewire 710 has a distal end configured to enter into the stent 800 from an outside thereof via the fenestration 811 on the stent 800. The pre-embedded guidewire 710 is configured to guide a branch guidewire to pass through the fenestration 811 from inside to outside of the stent 800. At that time, the connection between the pre-embedded guidewire 710 and the branch guidewire may be released. Thereafter, since the fenestration 811 is aligned with the branch vessel, the branch guidewire may be pushed further by the operator to enter the branch vessel. Thereafter, the branch stent may be guided into the branch vessel by the branch guidewire. In addition, in this embodiment, the distal end of the pre-embedded guidewire passes through the push-rod.

In this embodiment, referring to FIG. 1, the proximal end of the delivery device 1000 is provided with a second locking assembly 720. Specifically, the second locking assembly 720 is arranged on the rear slideway 650 and configured to lock the movement of the pre-embedded guidewire 710.

Specifically, referring to FIG. 7, the rear fastener 640 defines therein a hollow rear sliding passage 651 extending in an axial direction. The rear sliding passage 651 communicates with the delivering gap 210, and the pre-embedded guidewire 710 enters the rear sliding passage 651. The second locking assembly 720 includes a wire fastener 721, a fixed annular protrusion 722 secured on the rear slideway 650 and a blocking member 723 secured inside the rear sliding passage 651. The pre-embedded guidewire 710 enters the rear sliding passage 651 from a gap between the blocking member 723 and the wire fastener 721. Herein, the wire fastener 721 and the fixed annular protrusion 722 may be movably connected by a threaded connection. When the pre-embedded guidewire 710 is pressed tightly by the wire fastener 721 onto the blocking member 723, the movement of the pre-embedded guidewire 710 is locked, such that the pre-embedded guidewire 710 is prevented from being pulled to an improper position by mistake.

A delivery system for controlling the release of a stent in a stepwise manner is provided according to an embodiment of the present disclosure. As shown in FIGs. 1 to 4, and 9 to 11, the delivery system 2000 for controlling the release of a stent in a stepwise manner includes a stent 800 and a delivery device 1000. The stent 800 includes a tubular membrane 810 and an annular support frame 820. The delivery device 1000 includes a core assembly 100, an outer sheath 200 and a stent restraining assembly 400. The outer sheath 200 is hollow, and is mounted around an outer periphery of the core assembly 100. A delivering gap 210 is defined between the outer sheath 200 and the core assembly 100. The delivering gap 210 has a distal end for receiving the folded stent 800. When the stent 800 is in a partly released state, the stent restraining assembly 400 makes the released part of the stent 800 be partly unfolded, so as to restrain an outer diameter of the released part of the stent 800, and makes the stent 800 be fully unfolded when the stent 800 is in a completely released state.

In this embodiment, a plurality of connecting members 830 are provided on the tubular membrane 810 which extend from a proximal end of the tubular membrane 810 towards a distal end of the tubular membrane 810. The connecting members 830 are arranged in at least two columns which are spaced apart in a circumferential direction. The connecting members 830 may be flexible rings. One to six connecting member(s), preferably three to five connecting members may be provided in a column. The connecting members arranged in a column in the axial direction may prevent the stent from being shortened when the delivery device restraining assembly is used. When the stent 800 is in the partly released state, the stent restraining assembly 400 is controlled to bound at least two columns of the connecting members to enable the stent 800 to be partly unfolded. When the stent 800 is in the completely released state, the stent restraining assembly 400 releases the bound to the connecting members 830, so as to enable the stent to be fully unfolded.

A fenestration 811 is provided on the tubular membrane 810. When the stent 800 is in the partly released state, the fenestration 811 is located on the released part of the tubular membrane. The specific structure of the stent 800 in this embodiment has been discussed in detail in a co-pending Chinese patent application No. 201711483955.7 early filed, the disclosed content of which is incorporated herein by reference.

In this embodiment, the stent restraining assembly 400 includes at least one control wire 410. The control wire 410 enters the delivering gap 210 through a proximal end of the delivering gap 210 and extends to a distal end of the delivering gap 210. The control wire 410 has a distal end bounding the released part of the stent 800 circumferentially. The control wire 410 ties at least two columns of the connecting members to restrain the stent 800 to be partly unfolded circumferentially.

In addition, the delivery device further includes a control handle 300 connected with the outer sheath 200. The control handle 300 controls the outer sheath 200 to move axially relative to the core assembly 100, so as to enable the folded stent 800 to be in a partly released state or in a completely released state. Detail descriptions of the delivery device have been provided above in the aforementioned embodiment, and will not be described here again.

It should be noted that each embodiment in this specification is described in a progressive manner and focuses on the differences from the other embodiments, and the same parts between those embodiments may be referred to each other. With respect to the embodiments for device, since it is substantially similar to the embodiments for method, the description therefor is relatively simple, and reference may be made to parts of the description in the embodiments of method.

The above disclosure merely describes preferred embodiments of the present disclosure without intention to limit the scope of the present disclosure, and thus equivalent changes made in accordance with the claims fall within the scope of the present disclosure.

## Claims

1. A delivery device for controlling the release of a stent in a stepwise manner, which is configured to deliver a stent and release the stent in a stepwise manner, **Characterized in that**, the delivery device comprises:
a core assembly;
an outer sheath, which is hollow and mounted around an outer periphery of the core assembly, with a delivering gap defined between the outer sheath and the core assembly, wherein the delivering gap has a distal end for receiving a folded stent; and
a stent restraining assembly, configured to enable the stent to be partly unfolded so as to restrain an outer diameter of a released part of the stent when the stent is in a partly released state and to enable the stent to be fully unfolded when the stent is in a completely released state.

2. The delivery device for controlling the release of a stent in a stepwise manner according to claim 1, wherein the delivery device further comprises a control handle connected with the outer sheath, and wherein the control handle controls the outer sheath to move axially relative to the core assembly so as to enable the folded stent to be in a partly released state or in a completely released state.

3. The delivery device for controlling the release of a stent in a stepwise manner according to claim 2, wherein the stent restraining assembly comprises at least one control wire, and the control wire enters the delivering gap through a proximal end of the delivering gap and extending to the distal end of the delivering gap, and wherein the control wire has a distal end configured to circumferentially restrain the released part of the stent.

4. The delivery device for controlling the release of a stent in a stepwise manner according to claim 3, wherein the stent restraining assembly further comprises a pull-tab secured to a proximal end of the control wire, the pull-tab is configured to apply a force to a proximal end of the delivery device so as to release the restraining of the control wire to the stent.

5. The delivery device for controlling the release of a stent in a stepwise manner according to claim 3, wherein the delivery device further comprises a first locking assembly configured to lock the movement of the stent restraining assembly to avoid releasing the restraining of the control wire to the stent unintentionally.

6. The delivery device for controlling the release of a stent in a stepwise manner according to any one of claims 2 to 5, wherein a sheath joint is secured to an outer periphery of the outer sheath, and the control handle comprises:
a support body, with the sheath joint provided therein, wherein when being subjected to an axial force, the sheath joint moves axially within the support body to drive the outer sheath to move axially;
a fixed handle, mounted outside a distal end of the support body and secured thereto; and
a sliding handle, mounted outside a distal end of the support body adjacent to a proximal end of the fixed handle, the sliding handle being rotatable at the outside of the support body so as to drive the sheath joint to move axially.

7. The delivery device for controlling release of stent in a stepwise manner according to claim 6, wherein an elongated hole is defined on the support body and extends in an axial direction, a tooth block is mounted outside the support body at a position corresponding to the elongated hole, the sheath joint comprises a joint body, and an abutment block and a distal protrusion provided on the joint body, the abutment block and the distal protrusion extend through the elongated hole and abut against a proximal end and a distal end of the tooth block, respectively, in such a way to limit an axial movement of the tooth block relative to the sheath joint, the sliding handle is provided with an internal thread, the tooth block is provided with an external thread engaging with the internal thread, and wherein when the sliding handle rotates, the sliding handle drives the sheath joint to move axially through the tooth block, the abutment block and the distal protrusion, and in turn drives the outer sheath to move axially.

8. The delivery device for controlling the release of a stent in a stepwise manner according to claim 6, wherein the sliding handle is axially slidable on the support body, an unlocking button is embedded in the fixed handle, the unlocking button extends to the side of the sliding handle with a hook, and the hook hooks the sliding handle in such a way that the sliding handle is arranged next to the fixed handle and prevented from axially sliding.

9. The delivery device for controlling the release of a stent in a stepwise manner according to claim 3, wherein the delivery device further comprises a push-rod, wherein the push-rod has a distal end disposed in the delivering gap, and the distal end of the push-rod is configured to abut against the stent when the control handle controls the outer sheath to axially move relative to the core assembly towards the proximal end, so as to prevent the stent from moving towards the proximal end of the delivering gap.

10. The delivery device for controlling the release of a stent in a stepwise manner according to claim 9, wherein the delivery device further comprises a support tube, wherein the support tube has a distal end disposed in the delivering gap, and the push-rod is disposed in the support tube.

11. The delivery device for controlling the release of a stent in a stepwise manner according to claim 9, wherein the push-rod defines therein a through passage extending in an axial direction thereof, and the control wire enters the through passage from a proximal end thereof and extends out of the through passage from a distal end thereof.

12. The delivery device according to any one of claims 2 to 5, wherein the delivery device further comprises a pre-embedded guidewire, wherein the pre-embedded guidewire enters the delivering gap through the proximal end of the delivering gap and extends to the distal end of the delivering gap, the pre-embedded guidewire has a distal end configured to enter an inside of the stent from an outside thereof via a fenestration defined on the stent, and the pre-embedded guidewire is configured to guide a branch guidewire to extend out of the stent via the fenestration of the stent.

13. The delivery device according to claim 12, wherein a second locking assembly is provided at a proximal end of the delivery device and configured to lock the pre-embedded guidewire to be prevented from moving.

14. A delivery system for controlling the release of a stent in a stepwise manner, **Characterized in that**, the delivery system comprises a stent and a delivery device, wherein the stent comprises a tubular membrane and an annular support frame, and the delivery device comprises:
a core assembly;
an outer sheath, which is hollow and mounted around an outer periphery of the core assembly, with a delivering gap defined between the outer sheath and the core assembly, wherein the delivering gap has a distal end receiving the folded stent; and
a stent restraining assembly, configured to restrain a released part of the stent to be partly unfolded so as to restrain an outer diameter of the released part of the stent when the stent is in a partly released state, and to enable the stent to be fully unfolded when the stent is in a completely released state.

15. The delivery system according to claim 14, wherein a plurality of connecting members are provided on the tubular membrane and extend from a proximal end of the tubular membrane towards a distal end of the tubular membrane, and the plurality of connecting members are arranged in at least two columns which are circumferentially arranged spaced apart.

16. The delivery system according to claim 15, wherein the stent restraining assembly is controlled to bound at least two columns of the connecting members together to restrain the stent to be partly unfolded when the stent is in the partly released state, and the stent restraining assembly releases the bound to the connecting members so as to make the stent be fully unfolded when the stent is in the completely released state.

17. The delivery system according to any one of claims 14 to 16, wherein a fenestration is provided on the tubular membrane, and the fenestration is located on a released part of the tubular membrane when the stent is in the partly released state.

18. The delivery system according to any one of claims 14 to 16, wherein the stent restraining assembly comprises at least one control wire which enters the delivering gap through a proximal end of the delivering gap and extending to the distal end of the delivering gap, and the control wire has a distal end restraining the released part of the stent circumferentially.

19. The delivery system according to any one of claims 14 to 16, wherein the delivery device further comprises a control handle connected with the outer sheath, the control handle controls the outer sheath to move axially relative to the core assembly so as to enable the folded stent to be in a partly released state or in a completely released state.
